# EUROPÄISCHE PATENTSCHRIFT

(11) **EP 3 009 469 B1**
(45) Veröffentlichungstag und Bekanntmachung des Hinweises auf die Patenterteilung: **25.08.2021**
(21) Anmeldenummer: 15002970.0
(22) Anmeldetag: 19.10.2015
(51) Int. Cl.: C08J 7/04, C08J 7/043, C12P 1/00

(54) **VERBUND MIT VERBESSERTER LACKHAFTUNG UND VERFAHREN ZU DESSEN HERSTELLUNG**
COMPOSITE WITH IMPROVED PAINT ADHESION AND METHOD FOR PRODUCING THE SAME
COMPOSITE PRESENTANT UNE ADHERENCE DE PEINTURE AMELIOREE ET SON PROCEDE DE FABRICATION

(30) Priorität: 17.10.2014 DE 102014221181
(43) Veröffentlichungstag der Anmeldung: 20.04.2016
(73) Patentinhaber: Fraunhofer-Gesellschaft zur Förderung der angewandten Forschung e.V., 80686 München (DE)
(72) Erfinder: Rischka, Klaus, 21255 Tostedt (DE); Yendry, Regina Corrales Urena, 28359 Bremen (DE); Brinkmann, Andreas, 27726 Worpswede (DE); Szardenings, Michael, 04299 Leipzig (DE)
(74) Vertreter: Eisenführ Speiser

(56) Entgegenhaltungen:
- WO-A1-2013/186392
- DE-A1-102005 045 770
- US-A- 5 015 677
- DATABASE WPI Week 197436 Thomson Scientific, London, GB; AN 1974-63920V XP002754891, & JP S49 30512 B (SHIRAISHI H) 13. August 1974 (1974-08-13)
- None

## Beschreibung

Die Erfindung betrifft einen Verbund, umfassend ein Kunststoffsubstrat oder ein Kohlenstoffsubstrat und einen Lack und eine zwischen Kohlenstoffsubstrat und Lack angeordnete aminosäurehaltige Zwischenschicht. Die Erfindung betrifft ferner die Verwendung einer aminosäurehaltige Zwischenschicht zur Verbesserung der Haftung zwischen einem Lack und einem Kunststoffsubstrat oder einem Kohlenstoffsubstrat sowie ein Verfahren zur Herstellung eines Verbundes aus Kunststoffsubstrat oder Kohlenstoffsubstrat mit einem Lack, wobei die Haftung des Lacks verbessert ist.

Die Vorbehandlung von Oberflächen ist ein wichtiger Prozess, um Probleme beim Lackieren zu verringern und die Qualität zu gewährleisten Aufgabe der Vorbehandlung ist es, den zu lackierenden Untergrund für die Lackierung vorzubereiten. Dieses ist notwendig, um bei der Lackierung selbst z.B. sowohl einen guten Verlauf als auch nach der Aushärtung eine gute Haftung und/oder einen optimalen Glanz des Lackes zu erzeugen. Es werden nahezu alle Materialien (Metalle, Kunststoffe sowie mineralische Untergründe) vorbehandelt. Folgende Oberflächeneigenschaften werden beispielsweise für eine Lackierung angepasst und verändert: Oberflächenrauigkeit, Oberflächenhärte, Oberflächenenergie, Oberflächenzusammensetzung, Penetrationsvermögen, Entfernung von Verunreinigungen, Entfernung von Konversionsschichten oder Alterungsschichten der Oberfläche. Vorbehandlungsverfahren können dabei mechanischer (Schleifen, Sandstahlen), physikalischer, (Coronabeflammung, Plasmaverfahren) oder chemischer (Beizen, Fluorierung) Art sein.

Die Vorbehandlung von Kunststoffe stellt dabei aufgrund der Sensibilität vieler Kunststofftypen eine besondere Herausforderung dar. Ihre teilweise niedrige Temperaturbeständigkeit und Schmelzbarkeit verhindert, dass thermische Prozesse eingesetzt werden können, da es zur Verformungen von Bauteilen bis hin zur pyrolytischen Zersetzung kommen kann. Weiter sind mechanische Vorhandlungsverfahren nicht beliebig einsetzbar, da eine hohe Elastizität Schleifen und Strahlverfahren nicht zulässt oder kein homogener Abtrag der Oberfläche möglich ist. Hierbei kann es zur Inkooperation von Strahl- und Schleifmittel in der Kunststoffoberfläche kommen. Weiter ist der Einsatz von beliebigen Bad- oder Tauchverfahren eingeschränkt, da viele Lösemittel Kunststoffe angreifen, diese z.T. Lösen oder es zu Rissen in Bauteilen aufgrund von Spannungen kommen kann. Deshalb besteht hier eine hohe Nachfrage und Forschungsbedarf nach neuen möglichst schonenden Prozessen, welche eine entsprechende Oberfläche nicht schädigen.

Beispiele für Vorbehandlungsverfahren von Kunststoffoberfläche zur anschließenden Lackierung sind:
- Schleifen
- Alkalische Reinigung
- Coronabeflammung
- Niederdruck- und Atmosphärendruckplasma
- Fluorbehandlung
- CO₂ Strahlen
- Silikatisierung

Die meisten Vorbehandlungsverfahren von Kunststoffen haben den Nachteil, dass sie apparativ aufwendig sind, spezielle Schutzmaßnahmen erfordern oder aufwendige Reinigungsschritte.

In der WO 2013/186392 A1 wird ein Verfahren zur Aktivierung einer Oberfläche beschrieben, bei dem eine Oxidoreduktase eingesetzt wird, die aber bevorzugt vollständig von der Oberfläche des Substrates wieder entfernt wird.

Daher war es Aufgabe der vorliegenden Erfindung, eine Möglichkeit anzugeben, mittels derer eine verbesserte Anhaftung von Lack gegenüber für diesen Lack im Regelfall problematischen Oberflächen wie Kunststoffe oder Kohlenstoffsubstrate gewährleistet werden kann.

Diese Aufgabe wird gelöst durch einen Verbund, umfassend ein Kunststoffsubstrat oder ein Kohlenstoffsubstrat und einen Lack, wobei zwischen dem Kunststoffsubstrat und dem Lack eine aminosäurehaltige Zwischenschicht angeordnet ist, wobei die aminosäurehaltige Zwischenschicht eine Dicke von 1-10 nm besitzt und Kupfer umfasst.

Ein Kunststoffsubstrat im Sinne der vorliegenden Erfindung ist ein Polymer, aus repetitiven, kohlenstoffhaltigen Untereinheiten. Dabei sind grundsätzlich alle Arten von Polymeren denkbar, wie z.B. Mischpolymere oder Propfpolymere.

Ein Kohlenstoffsubstrat im Sinne der vorliegenden Erfindung ist ein Substrat, dass aus elementarem Kohlenstoff besteht. Dabei kann ein solches Substrat im Extremfall sehr dünn, bis zu einer Atomdicke (Graphen) sein.

Ein Lack im Sinne der vorliegenden Erfindung ist ein flüssiger oder pastenförmiger oder pulverförmiger pigmentierter Beschichtungsstoff, der, auf einen Untergrund aufgebracht, eine deckende Beschichtung mit schützenden, dekorativen oder anderen speziellen technischen Eigenschaften ergibt. Hierzu wird auch auf die Normen DIN EN 13523-0 (1999-00) und DIN EN 927 verwiesen.

Umfasst von der Definition für "Lack" im Sinne der vorliegenden Erfindung ist auch ein nicht-pigmentierter Lack, also ein Klarlack.

Zur Definition von "Beschichtungsstoff" wird im Zweifelsfall auf die DIN 5632-1 (1995-1200), DIN 55690 (1988-001-00) und DIN 55928-8 (1994-07-00) verwiesen, wobei die letztgenannte Norm bei noch immer verbleibenden Zweifeln ausschlaggebend ist.

"Aminosäurehaltig" im Sinne der vorliegenden Erfindung ist eine Schicht dann, wenn in ihr einzelne Aminosäuren oder Aminosäuren in Form von Poly-Aminosäuren nachgewiesen werden können. Dabei können Poly-Aminosäuren (Polypeptide, Proteine) auch aus verschiedenen Aminosäuren bestehen.

Es hat sich überraschenderweise herausgestellt, dass es möglich ist, mit Hilfe des Enzyms Laccase Polymeroberflächen in wässigem Medium so zu aktivieren, dass die Benetzbarkeit mit polaren Mitteln, insbesondere Wasser verbessert wird. Dabei ist der Aktivierungsschritt überraschend einfach durchzuführen, es genügt eine verhältnismäßig kurze Inkubation mit einer wässrigen Laccaselösung, um im Regelfall hydrophobe Oberflächen hydrophiler zu machen und so für eine nachfolgende Anhaftung von Lacken vorzubereiten.

Dabei hat sich überraschenderweise herausgestellt, dass die vorgenommene Aktivierung dauerhaft ist. Anders als bei Verfahren, bei denen reaktive (aktivierende) Gruppen direkt kovalent an den Kunststoff gebunden werden, führt die erfindungsgemäße Aktivierung durch Behandlung mit Laccase zu einem schichtartigen Aufbau einer aminosäurehaltigen Schicht. Dadurch, dass es anscheinend keine kovalente Anbindung an einzelne Atome des Substrates gibt. ist die Wahrscheinlichkeit deutlich verringert, dass die "Aktivierung" durch Umlagerung in das Innere des Substrates verlagert wird.

Hierbei ist zu beachten, dass die erfindungsgemäß vorzusehende aminosäurehaltige Zwischenschicht nicht flächendeckend sein muss. Ist aber erfindungsgemäß bevorzugt, dass sie mindestens 40%, bevorzugt mindestens 45%, weiter bevorzugt mindestens 50% und besonders bevorzugt mindestens 60% der mit Laccase behandelten Oberfläche bedeckt.

Die Erfinder gehen davon aus, dass die aminosäurehaltige Schicht Laccase oder Teile der Laccase umfasst. Dabei sind die Beschichtungsbestandteile überraschend stabil, da sie weder wasser- noch deterganzlöslich sind.

Laccasen im Sinne der vorliegenden Erfindung sind Oxireduktasen aus den Klassen EC 1.10.3.2 (Laccasen) nach der Klassifizierung nach dem Integrated Relational Enzyme Database des Swiss Institute of Bioinformatics (SIB).

Wie schon oben angedeutet, ist die Behandlung mit Laccase eine einfach durchzuführende Vorbehandlung, die wenig spezielle Maßnahmen benötigt. Grundsätzlich ist es möglich, eine entsprechende Aktivierungslösung (Laccase-Lösung) *in situ* für viele Anwendungsfälle einzusetzen Somit stellt die Erfindung neben einer einfachen Handhabbarkeit gegenüber bestehenden Aktivierungstechniken, zum Beispiel mittels Plasma oder Lösungsmittel, auch eine kostengünstige und umweltschonende Methode dar.

Bevorzugt ist ein erfindungsgemäßer Verbund, wobei die aminosäurehaltige Zwischenschicht eine Dicke von 2 - 7 nm, weiter bevorzugt 3 - 6 nm besitzt.

Wie bereits oben angedeutet, zeigt die durch die Laccase-Vorbehandlung entstehende Zwischenschicht auf dem Substrat das Verhalten von einem Monolayer. Dieses ist besonders gut geeignet, eine entsprechende Haftverbesserung zwischen Substraten und Lack zu gewährleisten.

Erfindungsgemäß ist ein entsprechender Verbund, wobei die Zwischenschicht Kupfer umfasst.

Laccase ist ein kupferhaltiges Enzym, beim Entstehen der Zwischenschicht lässt sich bei entsprechender Verfahrensausgestaltung (vgl. z. B. Beispiel 1) auch Kupfer nachweisen.

Bevorzugt im Sinne der vorliegenden Erfindung ist, dass die aminosäurehaltige Zwischenschicht 2 bis 10 Atom-% Stickstoff, bevorzugt 3 bis 7 Atom-% Stickstoff und besonders bevorzugt 3,5 bis 6 Atom-% Stickstoff umfasst und/oder 5 bis 20 Atom-% Sauerstoff, bevorzugt 6 bis 13 Atom-% Sauerstoff und besonders bevorzugt 7 bis 11 Atom-% Sauerstoff umfasst, wobei diese Werte bestimmt werden mittels XPS und bezogen sind auf die Gesamtmenge aller mittels XPS erfassbaren Atome in der Zwischenschicht.

Bevorzugt ist ein erfindungsgemäßer Verbund, wobei die Zwischenschicht mindestens eine, mindestens zwei, mindestens drei oder bevorzugt alle Aminosäuren umfasst, ausgewählt aus der Gruppe bestehend aus Glycin, Alanin, Prolin, Asparagin, Valin, Leucin und Threonin.

Sofern sich eine, mehrere oder alle der genannten Aminosäuren in der Zwischenschicht befinden, weist dies darauf hin, dass eine optimierte Verfahrensführung bei der Aktivierung des Substrates gewählt wurde.

Besonders vorteilhaft ist die vorliegende Erfindung für Substrate, die hydrophob und/oder oleophil sind.

Hydrophob bedeutet im Sinne der vorliegenden Erfindung, dass die Substrate (unbeschichtet) über einen statischen Wasserrandwinkel von größer 65° verfügen. Der statische Wasserrandwinkel (auch Kontaktwinkel oder Benetzungswinkel) wird im Zweifelsfall nach DIN 55660-2:2011-12 bestimmt.

Dementsprechend ist bevorzugt, im Sinne der vorliegenden Erfindung, dass das Substrat ohne Beschichtung und über einen statischen Wasserrandwinkel von ≥65°, bevorzugt ≥ 70° verfügt.

Bevorzugt im Sinne der vorliegenden Erfindung ist, dass das Substrat ausgewählt aus der Gruppe bestehend aus Graphit; Graphen; Polyolefinen, insbesondere Polyethylen und Polypropylen; Polystyrol; cycloolefinische Polymere, insbesondere TOPAS; Polycarbonat sowie deren Copolymere, Mischungen aus diesen Polymeren und deren Mischpolymere.

Hierbei ist zu beachten, dass sich die Kohlenstoffsubstrate im Sinne der vorliegenden Erfindung häufig hinsichtlich ihrer Oberflächeneigenschaften von den Kunststoffsubstraten unterscheiden. Umso überraschender ist es, dass die vorliegende Erfindung auch auf Kohlenstoffsubstrate anwendbar ist.

Bevorzugt im Sinne der vorliegenden Erfindung ist, dass der erfindungsgemäße Verbund einen Lack umfasst, der mittels eines hydrophilen Lösemittels, insbesondere mittels Wasser, aufgebracht wird.

Bevorzugt im Sinne der vorliegenden Erfindung ist, dass der Lack ausgewählt ist aus der Gruppe bestehend aus Polyurethanlack, Polyesterlack, Epoxidlack und Acrylatlack.

Diese Lacke eigenen sich besonders gut für den erfindungsgemäßen Verbund.

Bevorzugt im Sinne der vorliegenden Erfindung ist ein erfindungsgemäßer Verbund, wobei die Haftkraft zwischen Substrat und Lack gegenüber einem gleichen Schichtverbund ohne die Zwischenschicht um wenigstens 20 %, bevorzugt wenigstens 25 %, weiter bevorzugt wenigstens 30 % erhöht ist.

Überraschenderweise hat es sich gezeigt, dass es zu einer erheblichen Haftkraftverbesserung durch die vorliegende Erfindung kommen kann.

Teil der Erfindung ist auch die Verwendung einer aminosäurehaltigen Zwischenschicht wle oben definiert zur Verbesserung der Haftung zwischen einem Lack, bevorzugt wie oben als bevorzugt beschrieben und einem Kunststoffsubstrat oder Kohlenstoffsubstrat, ebenfalls bevorzugt wie oben als bevorzugt beschrieben.

Diese Verwendung stellt einen wesentlichen Kern der vorliegenden Erfindung dar.

Teil der Erfindung ist auch ein Verfahren zur Herstellung eines erfindungsgemäßen Verbundes, umfassend die Schritte
a) Bereitstellen eines Kunststoffsubstrates oder eines Kohlenstoffsubstrates, bevorzugt wie oben als bevorzugt beschrieben,
b) Bereitstellen einer Laccase-Lösung,
c) Inkubieren wenigstens eines Teils der Oberfläche mit der Laccase-Lösung,
d) Bereitstellen eines Lackes, bevorzugt wie weiter oben als bevorzugt beschrieben und
e) Auftragen eines Lackes wenigstens auf einen Teil der in Schritt c) behandelten Oberfläche des Substrates.

So kann der erfindungsgemäße Verbund effektiv hergestellt werden.

Bevorzugt ist es, dass die Laccase-Lösung 0,01 mg/ml bis 1,6 mg/ml Laccase enthält, weiter bevorzugt 0,1 mg/ml bis 1 mg/ml und besonders bevorzugt 0,4 bis 0,6 mg/ml.

Bevorzugt ist, dass die Einwirkungszeit auf die Substratoberfläche durch die Laccase-Lösung im Bereich von 1 bis 100 Minuten, bevorzugt 5 bis 60 Minuten liegt.

### Beisplele

### Beispiel 1: Oberflächenmodifikation durch Laccase

### Probenvorbereitung:

Kommerziell erhältliches Polystyrol, Polypropylen, Polyethylen, HOPG (Highly Ordered Pyrolytic Graphite) wurden als Substrat verwendet. Essigsäure, Natriumacetat, Natriumchlorid wurden als hoch reine Reagenzien verwendet. Die Laccaselösung wurde mit *trametes versicolor* Laccase (ASA Spezial enzyme GmbH) (Enzymaktivität 10.000 U/g) in Acetatepuffer 2 M, pH 4,75 mit einer Konzentration von 0,1 mg/ml hergestellt. Hieraus resultierte eine Aktivitätskonzentration von 1 U/ml. Die Lösung wurde unter Verwendung von deionisiertem Wasser (Across Organics) zubereitet.

Die so zubereitete Laccaselösung wurde in einer Menge von 100 µl/cm² in Kontakt mit der Oberfläche des Substrates gebracht. Dabei wurde die Laccase auf die Polymeroberflächen für eine Stunde aufgebracht. Das Aufbringen erfolgte durch Tropfen. Grundsätzlich ist es auch möglich, z.B. im Falle von dreidimensionalen strukturierten Oberflächen das Substrat in die Laccaselösung zu tauchen.

Nach dieser Behandlung wurde das Substrat mindestens dreimal mit deionisiertem Wasser gewaschen. Der Flüssigkeitsfilm wurde mit Pressluft von der Oberfläche geblasen und die Proben bei Raumtemperatur getrocknet.

### Ergebnisse:

Die Wasserkontaktwinkel wurden unter Verwendung eines Goniometers (OCA15 Plus, Data Physics Instruments, Germany) mittels statischer Tropfentechnik und HPLC grade Wasser (Across Organics) als Probeflüssigkeit gemessen. Das Volumen der Tropfen wurde konstant auf 10 µl auf jede Messung gehalten. Die Kontaktwinkel in der nachfolgenden Tabelle 1 sind Durchschnittswerte von wenigstens 9 separaten Tropfenmessungen auf verschiedenen Bereichen der behandelten Substratoberfläche.

**Tabelle 1**

| Substrat | Statischer Wasserkontaktwinkel vor Behandlung | Statischer Wasserkontaktwinkel nach Behandlung |
|---|---|---|
| HOPG Substrat | 79,4 ± 1,2° | 54,3 ± 1,3° |
| Polyethylen | 102,8±3° | 60,5±4,6° |
| Polypropylen | 100,6±4° | 55,3±2.8° |
| Polystyrol | 93±3° | 77,3±2° |

### Beispiel 2: Veränderung des Kontaktwinkels abhängig von der Laccase-Konzentration

Wasserkontaktwinkelmessungen wurden nach Behandlung mit Laccase-Konzentration von 0,01 mg/ml, 0,1 mg/ml, 0,5 mg/ml, 0,7 mg/ml und 1,6 mg/ml durchgeführt. Das verwendete Substrat war Polystyrol. Die Herstellung der Laccase-Lösung und die Behandlung des Substrates erfolgte analog zu Beispiel 1, wobei Wasserkontaktwinkelveränderungen nach 1, 10, 60, 100 und mehr als 1000 Minuten an Wirkzeit gemessen wurden.

Als Ergebnis stellte sich heraus, dass es eine Tendenz gibt, dass der Wasserkontaktwinkel sich schneller verringert, wenn höhere Laccase-Konzentrationen eingesetzt werden. Überraschenderweise allerdings ist dieser Effekt bei Konzentrationen von höher als 0,5 mg/ml ab 10 Minuten Einwirkzeit weitgehend vernachlässigbar: Nach dieser Zeit ist bei den entsprechenden Konzentrationen ein Wasserkontaktwinkel von etwa 70° erreicht.

### Beispiel 3: Veränderung der Atomprozente von Sauerstoff und Stickstoff durch Laccase-Einwirkung

Die Veränderung der Anteile von Sauerstoff und Stickstoff an der Oberfläche des Substrates aufgrund von Laccase-Einwirkung wurden mittels XPS bestimmt.

XPS-Spektren wurden unter Verwendung des Kratos Ultra mit folgenden Aufnahmeparametern aufgenommen:
Basisdruck: 4*10⁻¹⁰ hPa, Probenneutralisation durch Anwendung von Elektronen mit geringer Energie (< 5 eV), Hybridmodus (elektrostatische und magnetische Linsen werden verwendet), Abgabewinkel der Elektronen 0°, Passenergie 20 eV (bzw. 40 eV für N1s-Spektren) für hochauflösende Spektren und 160 eV in Überblicksspektren, Anregen der Photoelektronen erfolgte mittels monochromatischer AlK-*α*-Strahlung. Der Analysebereich ist elliptisch geformt mit Hauptachsen von 300 µm x 700 µm.

Die Steigerung von Sauerstoff- und Stickstoffgehalte abhängig von der Behandlungszeit auf der Oberfläche bei konstanter Konzentration der Laccase-Lösung von 0,1 mg/ml in Acetatpuffer, hergestellt analog zu Beispiel 1) wurde mittels XPS gemessen. Hierzu wurde zu den in der Tabelle angegebenen Zeiten jeweils die Laccase-Behandlung abgebrochen, nachfolgend analog zu Beispiel 1) getrocknet, gereinigt und dann die XPS-Messung durchgeführt.

Tabelle 2 zeigt, dass die Steigerung der Konzentration von Sauerstoff- und Stickstoff-Spezies nach 30 Minuten nicht mehr wesentlich steigt, wobei im Falle von Stickstoff ein langfristiger leichter Anstieg noch beobachtbar ist.

**Tabelle 2**

| Zeit (min.) | at-% O (1s) | at-% N (1s) |
|---|---|---|
| 0 | 0,1 | 2 |
| 1 | 7,58 | 2,52 |
| 5 | 8,34 | 2,80 |
| 10 | 8,14 | 3,20 |
| 20 | 8,40 | 3,74 |
| 30 | 10,27 | 4,0 |
| 60 | 10,46 | 4,8 |
| 1440 | 10,9 | 5,9 |

Angegeben ist die Veränderung der Atomprozent gegenüber dem Substrat für die Anteile der mittels XPS messbaren Atome. Das verwendete Substrat war Polystyrol.

Unter den gleichen Bedingungen wurde die Veränderung der Anteile von Stickstoff und Sauerstoff für das Substrat Polyethylen gemessen. Die Ergebnisse finden sich in der Tabelle 3:

| Zeit | at-% N (1s) | at-% O (1s) |
|---|---|---|
| 0 | 0 | 2,04 |
| 1 | 2,955 | 7,525 |
| 5 | 3,43 | 7,285 |
| 10 | 3,13 | 8,01 |
| 20 | 3,955 | 8,64 |
| 30 | 3,895 | 9,16 |
| 60 | 5,48 | 12,93 |

### Beispiel 4: Untersuchung der Substratoberfläche nach Behandlung mit Laccase-Lösung.

Nach einer Behandlung wie in Beispiel 1 beschrieben wurde (mit Laccase) modifiziertes Polystyrol sowie unmodifiziertes Polystyrol und Pulver aus der eingesetzten Laccase mittels Flugzeitmassenspektrometrie (ToF-SIMS) analysiert. Es wurde ein ToF-SIMS IV-Instrument (Ion ToF Ltd., Germany) verwendet. Die Oberfläche der untersuchten Proben wurde mittels primärer Ionen aus einer gepulsten Bi-lonenquelle beschossen. Durch die Einwirkung dieser primären Ionen werden sowohl neutrale Fragmente als auch positiv als auch negativ geladene sekundäre Ionen von der Oberfläche emittiert. Die sekundären Ionen werden zu einem Flugzeitmassenanalysator beschleunigt. Durch präzise Messung der Massen der emittierten Fragmente können diese Fragmente identifiziert werden. Da alle sekundären Ionen parallel detektiert werden, ist dieses Verfahren sehr oberflächensensitiv. Das ToF-SIMS-Verfahren ist qualitativ. Die der Tabelle 4 zugrundeliegenden Spektren wurden unter Verwendung eines gepulsten Bi primären lonenstrahles (25 keV) aufgenommen, wobei der Strahl einer Fläche von 500 x 500 µm² betraf.

Die Laccasepulverspektren zeigen hauptsächlich Peaks, die mit Aminosäuren korrespondierten.

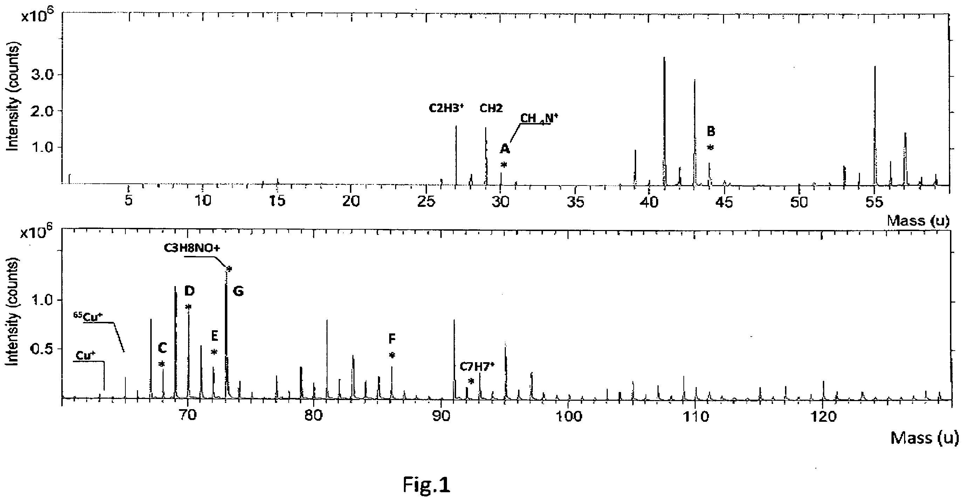

Fig. 1 zeigt das ToF-SIMS-Spektrum der mit Laccase-Lösung behandelten Polystyrol-Oberfläche.

Hier zeigen sich sowohl Signale für Kupfer als auch für Aminosäuren, die in der Figur mit den Buchstaben A bis G bezeichnet sind. Die korrespondierenden Aminosäuren sind in der Tabelle 4 aufgeführt. Das Hauptsignal von Polystyrol (C7H7+) ist auf der mit Laccase behandelten Oberfläche deutlich verringert gegenüber der unbehandelten Oberfläche.

**Tabelle 4**

| Positive Ionen-Peaks | Symbol | Fragment | Aminosäure |
|---|---|---|---|
| 30 | A | CH4N+ | Glycin |
| 44 | B | C2H6N+ | Alanin |
| 68 | C | C4H6N+ | Prolin |
| 70 | D | C4H8N+ | Asparagin |
| 72 | E | C4H10N+ | Valin |
| 86 | F | C5H12N+ | Leucin |
| 74 | G | C3H8NO+ | Threonin |

Als Ergebnis lässt sich feststellen, dass offensichtlich Reste der Laccase auf der Polystyrol-Oberfläche verblieben sind.

### Beispiel 5:

Eine Prepreg CFK Gewebe-Probe der Dicke 11 mm mit einen 1/4 Atlas Gewebeprepreg, einer 370 g/m² Epoxidharz Matrix (DGEBA, amingehärtet), welches im Laminataufbau: 0/90° mit Tenax® E HTS40-Fasern (aus Polyacrylnitril, HT), (12K) mit einen Faserdurchmesser 7 µm hergestellt wurde analog zu Beispiel 1 mit Laccase behandelt. Nachfolgend wurde ein Lack auf wässriger Polyurethanbasis (Hersteller, Firma Mankiewicz, Lack: ALEXSEAL® W) aufgebracht.

Nach Verfestigen der Polyurethan-Beschichtung (Lack) wurde eine Gitterschnittprüfung nach DIN EN ISO 1209 durchgeführt. Als Vergleichswert diente ein auf dieselbe Art beschichtetes CFK-Substrat, das keine Laccase-Vorbehandlung vor der Beschichtung erhalten hatte.

Analog zu dem vorbeschriebenen Versuch wurde eine mit Laccase behandelte Polypropylen-Probe wie in Beispiel 1 beschrieben mit der Polyurethan-Beschiohtung wie oben beschrieben versehen. Als Vergleichsprobe diente hier wiederum eine analog beschichtete Polypropylen-Probe, die nicht mit Laccase vorbehandelt wurde.

Nach der Beschichtung wurde die Haftkraft mit einem Stirnabzugsversuch nach EN ISO 4624:2014 durchgeführt. Das Ergebnis ergab für Polypropylen ohne Laccase-Vorbehandlung eine Haftkraft von 0,2 MPa, für Polypropylen mit Laccase-Vorbehandlung eine Haftkraft von 0,35 MPa. Dementsprechend konnte eine Zunahme der Haftung durch die Laccase-Vorbehandlung von 40 % erzielt werden.

Es zeigte sich eine deutlich verbesserte Haftung der Beschichtung für das Laccasevorbehandelte Substrat. Die analog gemessene Haftkraft des Verbundes mit CFK als Substrat (s.o.) ohne Laccase-Vorbehandlung betrug 2,43 MPa, des Verbundes mit Laccase-Vorbehandlung 3,33 MPa. Es konnte also eine Zunahme der Haftung um 37 % erzielt werden.

## Patentansprüche

1. Verbund, umfassend ein Kunststoffsubstrat oder ein Kohlenstoffsubstrat und einen Lack, wobei zwischen dem Kunststoffsubstrat und dem Lack eine aminosäurehaltige Zwischenschicht angeordnet ist, **dadurch gekennzeichnet, dass**
die aminosäurehaltige Zwischenschicht eine Dicke von 1 - 10 nm besitzt und
dass die Zwischenschicht Kupfer umfasst.

2. Verbund nach Anspruch 1, wobei die Zwischenschicht mindestens eine, mindestens zwei, mindestens drei oder bevorzugt alle Aminosäuren umfasst, ausgewählt aus der Gruppe bestehend aus Glycin, Alanin, Prolin, Asparagin, Valin, Leucin und Threonin.

3. Verbund nach einem der vorangehenden Ansprüche, wobei das Substrat ohne Beschichtung über einen statischen Wasserrandwinkel von ≥ 65°, bevorzugt ≥ 70° verfügt.

4. Verbund nach einem der vorangehenden Ansprüche, wobei das Substrat ausgewählt ist aus der Gruppe bestehend aus Graphit; Graphen; Polyolefinen, insbesondere Polyethylen und Polypropylen; Polystyrol; cycloolefinische Polymere, Polycarbonat sowie deren Copolymere, Mischungen aus diesen Polymeren und deren Mischpolymere.

5. Verbund nach einem der vorangehenden Ansprüche, wobei der Lack ausgewählt ist aus der Gruppe bestehend aus Polyurethanlack, Polyesterlack, Epoxidlack und Acrylatlack.

6. Verbund nach einem der vorangehenden Ansprüche, wobei die Haftkraft zwischen Substrat und Lack gegenüber einem gleichen Schichtverbund ohne die Zwischenschicht um wenigstens 20 %, bevorzugt wenigstens 25 %, weiter bevorzugt wenigstens 30 % erhöht ist.

7. Verwendung einer aminosäurehaltigen Zwischenschicht wie in einem der Ansprüche 1 oder 2 definiert zur Verbesserung der Haftung zwischen einem Lack, bevorzugt wie in Anspruch 5 definiert und einem Kunststoffsubstrat oder einem Kohlenstoffsubstrat, bevorzugt wie ein einem der Ansprüche 3 oder 4 definiert.

8. Verfahren zur Herstellung eines Verbundes nach einem der Ansprüche 1 bis 6, umfassend die Schritte:
a) Bereitstellen eines Kunststoffsubstrates oder ein Kohlenstoffsubstrates, bevorzugt wie ein einem der Ansprüche 3 oder 4 definiert,
b) Bereitstellen einer Laccase-Lösung,
c) Inkubieren wenigstens eines Teils der Oberfläche mit der Laccase-Lösung,
d) Bereitstellen eines Lackes und
e) Auftragen des Lackes wenigstens auf einen Teil der in Schritt c) behandelten Oberfläche des Substrates.

## Claims

1. Assembly comprising a plastics substrate or a carbon substrate and a coating material, there being disposed between the plastics substrate and the coating material an amino acid-containing interlayer, **characterized in that**
the amino acid-containing interlayer possesses a thickness of 1-10 nm and
**in that** the interlayer comprises copper.

2. Assembly according to Claim 1, the interlayer comprising at least one, two, at least three or preferably all amino acids selected from the group consisting of glycine, alanine, proline, asparagine, valine, leucine and threonine.

3. Assembly according to either of the preceding claims, the uncoated substrate possessing a static water contact angle of ≥ 65°, preferably ≥ 70°.

4. Assembly according to any of the preceding claims, the substrate being selected from the group consisting of graphite; graphene; polyolefins, more particularly polyethylene and polypropylene; polystyrene; cycloolefinic polymers, polycarbonate, and also copolymers thereof, mixtures of these polymers and copolymers thereof.

5. Assembly according to any of the preceding claims, the coating material being selected from the group consisting of polyurethane, polyester, epoxy and acrylate coating materials.

6. Assembly according to any of the preceding claims, the adhesive force between substrate and coating material being increased, relative to an identical layer assembly without the interlayer, by at least 20%, preferably at least 25%, more preferably at least 30%.

7. Use of an amino acid-containing interlayer as defined in either of Claims 1 and 2 for improving the adhesion between a coating material, preferably as defined in Claim 5, and a plastics substrate or a carbon substrate, preferably as defined in either of Claims 3 and 4.

8. Method for producing an assembly according to any of Claims 1 to 6, comprising the steps of:
a) providing a plastics substrate or a carbon substrate, preferably as defined in either of Claims 3 and 4,
b) providing a laccase solution,
c) incubating at least part of the surface with the laccase solution,
d) providing a coating material,
e) applying the coating material at least to a part of the substrate surface treated in step c).

## Revendications

1. Composite comprenant un substrat en matière plastique ou un substrat en carbone et une peinture, dans lequel une couche intermédiaire contenant un acide aminé est disposée entre le substrat en matière plastique et la peinture, **caractérisé en ce que**
la couche intermédiaire contenant de l'acide aminé possède une épaisseur de 1 - 10 nm, et
**que** la couche intermédiaire présente du cuivre.

2. Composite selon la revendication 1, dans lequel la couche intermédiaire comprend au moins un, au moins deux, au moins trois et de manière préférée tous les acides aminés choisis parmi le groupe constitué de glycine, alanine, proline, asparagine, valine, leucine et thréonine.

3. Composite selon l'une quelconque des revendications précédentes, dans lequel le substrat sans revêtement dispose d'un angle de contact avec l'eau statique ≥ 65°, de manière préférée ≥ 70°.

4. Composite selon l'une quelconque des revendications précédentes, dans lequel le substrat est choisi parmi le groupe constitué de graphite ; graphène ; polyoléfines, en particulier polyéthylène et propylène ; polystyrène ; polymères cyclooléfiniques, polycarbonate ainsi que leurs copolymères, mélanges composés desdits polymères et leurs copolymères.

5. Composite selon l'une quelconque des revendications précédentes, dans lequel la peinture est choisie parmi le groupe constitué de peinture de polyuréthane, peinture de polyester, peinture époxy et peinture acrylate.

6. Composite selon l'une quelconque des revendications précédentes, dans lequel la force d'adhérence entre le substrat et la peinture par rapport à un composite stratifié identique sans la couche intermédiaire est plus élevée d'au moins 20 %, de manière préférée d'au moins 25 %, de manière davantage préférée d'au moins 30 %.

7. Utilisation d'une couche intermédiaire contenant de l'acide aminé telle que définie dans l'une quelconque des revendications 1 ou 2 pour améliorer l'adhérence entre une peinture de manière préférée telle que définie dans la revendication 5 et un substrat en matière plastique ou un substrat en carbone de manière préférée tel que défini dans l'une quelconque des revendications 3 ou 4.

8. Procédé pour fabriquer un composite selon l'une quelconque des revendications 1 à 6, comprenant les étapes :
a) de fourniture d'un substrat en matière plastique ou d'un substrat en carbone, de manière préférée tel que défini dans l'une quelconque des revendications 3 ou 4,
b) de fourniture d'une solution à base de laccase,
c) d'incubation d'au moins une partie de la surface avec la solution à base de laccase,
d) de fourniture d'une peinture et
e) d'application de la peinture au moins sur une partie de la surface, traitée lors de l'étape c), du substrat.
